# EUROPÄISCHE PATENTSCHRIFT

(11) **EP 1 244 425 B1**
(45) Veröffentlichungstag und Bekanntmachung des Hinweises auf die Patenterteilung: **09.02.2005**
(21) Anmeldenummer: 01905640.7
(22) Anmeldetag: 03.01.2001
(51) Int. Cl.: A61K 7/48, A61K 35/02

(54) **VERFAHREN ZUR HERSTELLUNG EINES KOSMETISCH VERWENDBAREN EXTRAKTES AUS MEERESSCHLICK**
METHOD FOR PRODUCING A COSMETICALLY APPLICABLE MARINE MUD EXTRACT
PROCEDE DE PRODUCTION D'UN EXTRAIT DE BOUES MARINES A USAGE COSMETIQUE

(30) Priorität: 07.01.2000 DE 10000443
(43) Veröffentlichungstag der Anmeldung: 02.10.2002
(73) Patentinhaber: La Mer Cosmetics AG, 27478 Cuxhaven (DE)
(72) Erfinder: GOJNY, Paul, 27478 Cuxhaven (DE)
(74) Vertreter: Schildberg, Peter, Dr. Dipl.-Phys.
(86) Internationale Anmeldenummer: PCT/EP2001/000020
(87) Internationale Veröffentlichungsnummer: WO 2001/049254

(56) Entgegenhaltungen:
- WO-A-00/40255
- CH-A- 286 680
- DE-A- 2 461 161
- DE-A- 3 113 287
- DE-U- 29 919 044
- DATABASE WPI Week 199946 Derwent Publications Ltd., London, GB; AN 1999-543909 XP002166778 & JP 11 228344 A (HEIWA GENSHI KK)
- DATABASE WPI Week 199621 Derwent Publications Ltd., London, GB; AN 1996-207469 XP002166779 & RU 2 043 100 C (SHCHERBAK)
- DATABASE WPI Week 199845 Derwent Publications Ltd., London, GB; AN 1998-529660 XP002166780 & RU 2 107 504 C (SIMONYAN)
- DATABASE WPI Week 199740 Derwent Publications Ltd., London, GB; AN 1997-433322 XP002166781 & RU 2 073 999 C (TOLCHEV)

## Beschreibung

Die Erfindung betrifft ein Verfahren zur Herstellung eines kosmetisch verwendbaren Extraktes aus Meeresschlick.

Organextrakte und Pflanzenextrakte sind seit Jahren beliebte und nach Meinung von Fachleuten wirksame Additive insbesondere für kosmetische Erzeugnisse verschiedenster Art. Es ist bekannt balneologische Packungen und Masken auf der Basis von Meeresschlick einzusetzen. Höherwertige Produkte, wie beispielsweise kosmetische Cremes, Lotionen und kosmetische Flüssigkeiten, konnten wegen der hohen unlöslichen Anteile im Meeresschlick nicht hergestellt werden.

Ein Verfahren zur Herstellung eines Extraktes aus Nordseeschlick ist aus dem deutschen Patent 31 13 287 bekannt. Nachteilig an diesem Verfahren ist, dass es nur einen Teil der wirksamen Bestandteile des Nordseeschlicks erfaßt und daher nur begrenzte Wirksamkeit besitzt. Ebenso ist das Verfahren vergleichsweise aufwendig in seiner Durchführung.

Aus DE 3 221 502 A1 ist eine kosmetische Crememaske mit Meeresschlick und ein Verfahren zu ihrer Herstellung bekannt. Die kosmetische Crememaske weist 10 - 40 % getrockneten Meeresschlick auf. Als Grundlage für die Creme kann sowohl eine Öl-In-Wasser-Creme als auch eine Wasser-In-Öl-Creme verwendet werden. Der Meeresschlick wird derart ausgewählt, daß Mineralpartikel mit einer Größe von 50 - 100 µm enthalten sind. Zur Herstellung der Crememaske wird der Trockenschlick nach dem Emulgieren der Cremegrundlage in die noch warme Emulsion eingetragen und anschließend kalt gerührt. Die Maske dient zur Pflege und Massage. Gleichzeitig tritt eine kosmetische Wirkung durch die mit dem Trockenschlick eingebrachten Schlickinhaltsstoffe ein.

Das DATABASE WPI Week 19621 Derwent; AN 1996-207469 XP002166779 und RU 2 043 110 ist die Herstellung einer Substanz für ein Schlammbad bekannt. Bei dem Verfahren wird der Ausgangsschlamm auf 12% Feuchtigkeit eingetrocknet und mit 95%igem Ethanol bei PH 9,0 angefeuchtet. Der Schlamm wird mit Ethanol oder einer Mischung aus Ethanol mit einem nicht polaren Lösungsmittel extrahiert und nachfolgend unter Zugabe von 1 Vol.-% Öl angedickt.

Aus DATABASE WPI Week 199845 Derwent; AN 1998-529660 XP00216678 & RU 2 107 504 C ist die Herstellung einer biologisch aktiven Substanz für ein Schlammbad und bei der Herstellung von Parfüm und Kosmetika bekannt. Bei dem Verfahren wird eine Ethanolextraktion eines Ausgangsschlamms und ein Eindicken des Extraktes verwendet.

Aus RU 2 073 999 C ist die Herstellung eines therapeutischen Schlammextraktes bekannt, bei dem ein Ausgangsschlamm bei einem Feuchtigkeitsgehalt von 50-60% pulverisiert wird, um Algenstile aufzubrechen, und anschließend zweifach mit 90%igem Ethanol bei einer Temperatur von 30-50°C unter raschem Rühren 20-25 min extrahiert wird.

Aufgabe der zugrunde liegenden Erfindung ist es, ein Extrakt aus Meeresschlick und ein Verfahren zur Herstellung eines kosmetisch verwendeten Extraktes aus Meeresschlick bereitzustellen, das wesentlich bessere Ausbeuten erzielt und mit einem geringeren Aufwand durchführbar ist.

Überraschenderweise ergab sich, dass mit dem folgenden Rezepturbeispiel: sich sowohl die wasserlöslichen als auch die weiteren für eine kosmetische Wirkung besonders wichtigen Bestandteile des Meeresschlicks gleichzeitig gut extrahieren lassen.

Erfindungsgemäß wird das angegebene Extraktionsmittel mit zerkleinertem Schlick vermischt. Das Gemisch wird extrahiert, indem die Bestandteile 1 bis 3 Stunden bei 50 bis 70°C gerührt werden.

Anschließend wird das Gemisch filtriert.

| Rezepturbeispiel 4b | |
|---|---|
| Wasser | 5 - 15 Gewichts-Teile |
| Glyzerin | 35 - 40 Gewichts-Teile |
| Propylenglycol | 25 - 35 Gewichts-Teile |
| Ethanol | 15 - 25 Gewichts-Teile |
| Getrocknetes Schlickpulver | 45 - 55 Gewichts-Teile. |

Das so hergestellte Gemisch aus Extraktionsmittel und Schlickpulver wird bei 50 bis 70 °C für 1 bis 3 Stunden gerührt und anschließend filtriert. Das nach dem Filtrieren gewonnene Extrakt besitzt eine gelbliche Farbe, die eine farbneutrale Verarbeitung in anderen Produkten besonders gut ermöglicht.

Bevorzugt beträgt das Verhältnis zwischen getrocknetem Schlickpulver und Extraktionsmittel dem Gewicht nach 1:2. Dieses Mischungsverhältnis stellt eine zuverlässige Extraktion der in dem Schlick enthaltenen Bestandteile sicher.

In einer bevorzugten Weiterführung des Verfahrens für das Rezepturbeispiel 4b erfolgt in einem ersten Schritt das Abwiegen des Schlickpulvers, dem unter Rühren das Wasser zugegeben wird. Die Zugabe von Propylenglycol und Glyzerin erfolgt anschließend, wobei die Suspension für 1 Stunde unter langsamer Erwärmung bis 60 °C gerührt wird. Anschließend kühlt die Mischung auf Raumtemperatur ab. Nachfolgend wird Ethanol zugegeben und die Suspension für 2 Stunden gerührt.

Bevorzugt ruht das so gewonnene Schlickextrakt für 24 Stunden wodurch eine Trennung von Feststoffphase und Flüssigkeitsphase erreicht wird. Das so durchgeführte Verfahren zur Herstellung eines Extraktes aus Schlick kann schnell und kostengünstig durchgeführt werden.

Bevorzugt wird für die Filtration das Schlickextrakt zunächst grob gefiltert und anschließend fein gefiltert. Vorzugsweise wird für die Feinfiltration Filterpapier eingesetzt, insbesondere Zellulosefilterpapier.

Das nach dem erfindungsgemäßen Verfahren hergestellte Meeresschlickextrakt ist besonders gut geeignet, in kosmetischen Produkten verschiedenster Art verwendet zu werden. Insbesondere kann das Extrakt pur oder als Anteil von herkömmlichen Kosmetikprodukten verwendet werden. Besonders vorteilhaft ist seine Anwendung bei Problemhaut-Zuständen.

Dermatologische Untersuchungen haben ergeben, daß die Verwendung des so gewonnenen Extraktes aus Schlick zu einer deutlichen Verbesserung der Hautfeuchtigkeit beim Menschen führt. Versuchsreihen haben gezeigt, daß bei der Anwendung eines Präparats mit Meeresschlickextrakt im Unterarmbereich über einen Zeitraum von 4 Wochen eine deutliche Verbesserung der Hautfeuchtigkeit erreicht wird.

Das folgende Anwendungsbeispiel soll die Einzelheiten des erfindungsgemäßen Verfahrens näher erläutern.

Als Ausgangsmaterial wird Meeresschlick mit folgender ungefährer Zusammensetzung seiner mineralischen Bestandteile verwendet:
24 bis 56 % Sand,
70 bis 90 % Silt,
10 bis 22 % Ton.

Schlick mit dieser Zusammensetzung wird als "sandiger Schlick" bezeichnet. Neben den mineralischen Bestandteilen weist der sandige Schlick eine Vielzahl von makroskopisch wahrnehmbaren Lebewesen wie beispielsweise Sandwürmer, Herzmuscheln, Tellmuscheln und Pfeffermuscheln auf.

Dieser Meeresschlick und damit auch die darin enthaltenen Lebewesen werden zerkleinert und mit einer Extraktionsmittelmischung nach einer der oben angegebenen Rezepturbeispiele gemischt. In einem ersten Herstellungsverfahren werden die Bestandteile 1 bis 3 Stunden bei 50 bis 70°C gerührt und anschließend filtriert. Das Filtrat wird bis zu seiner Verwendung in kosmetischen Produkten in Aluminiumflaschen gefiillt und im Kühlschrank aufbewahrt.

Die vorliegende Erfindung gibt die Möglichkeit, die organischen, kosmetisch wirksamen Stoffe des Meeresschlicks auch in hochwertige kosmetische Produkte einzuarbeiten. Die oben genannten Extraktionsmittel eignen sich auch zur Anwendung auf andere natürliche Mischungen aus biologischen Abbauprodukten.

## Patentansprüche

1. Verfahren zur Herstellung eines kosmetisch verwendbaren Extraktes aus Schlick, insbesondere aus Meeresschlick, **dadurch gekennzeichnet, daß**
- der zerkleinerte makroskopische Lebewesen enthaltende Schlick in Form von getrocknetem Schlickpulver mit einem Extraktionsmittel aus 5 bis 15 Gewichts-Teilen Wasser, 35 bis 40 Gewichts-Teilen Glyzerin, 25 bis 35 Gewichts-Teilen Propylenglycol und 15 bis 25 Gewichts-Teilen Ethanol gemischt wird, wobei das getrocknete Schlickpulver 45 bis 55 Gewichts-Teile aufweist,
- und das Gemisch aus Extraktionsmittel und Schlickpulver bei 50 °C bis 70 °C für ungefähr 1 bis 3 Stunden gerührt und anschließend filtriert wird.

2. Verfahren zur Herstellung eines kosmetisch verwendbaren Extraktes aus Schlick, insbesondere aus Meeresschlick, **dadurch gekennzeichnet, daß** die folgenden Verfahrensschritte durchgeführt werden:
- Einwaage des getrockneten Schlickpulvers,
- Zugabe von Wasser und Verrühren von Wasser und Schlickpulver,
- Zugabe von Propylenglycol und Glyzerin,
- Verrühren der Suspension für etwa 1 Stunde unter langsamer Erwärmung auf etwa 60 °C,
- Abkühlen der Mischung auf Raumtemperatur,
- Zugabe von Ethanol und Rühren der Suspension für etwa 2 Stunden.

3. Verfahren nach Anspruch 2, **dadurch gekennzeichnet, daß**
- das Gemisch anschließend für etwa 24 Stunden ruht, wodurch eine Trennung von Feststoffphase und Flüssigkeitsphase erreicht wird.

4. Verfahren nach Anspruch 3, **dadurch gekennzeichnet, daß**
- die abgesetzte Feststoffphase des Gemisches erst grob und anschließend fein filtriert wird.

5. Verfahren nach Anspruch 4, **dadurch gekennzeichnet, daß** zur Feinfiltration Filterpapier, insbesondere Zellulosefilterpapier eingesetzt werden.

6. Verfahren nach einem der Ansprüche 1 bis 5 , **dadurch gekennzeichnet, dass** das Verhältnis zwischen getrocknetem Schlickpulver und Extraktionsmittel dem Gewicht nach etwa 1:2 beträgt.

## Claims

1. Method for manufacturing an extract for cosmetic use made from mud, in particular from marine mud, **characterised in that**
- the mud containing comminuted macroscopic organisms in the form of dried mud powder is mixed with an extractant made up of 5 to 15 parts by mass of water, 35 to 40 parts by mass of glycerin, 25 to 35 parts by mass of propylene glycol and 15 to 25 parts by mass of ethanol, the dried mud powder comprising 45 to 55 parts by mass,
- and the mixture made up of the extractant and the mud powder being stirred at 50°C to 70°C for approximately 1 to 3 hours and subsequently filtered.

2. Method for manufacturing an extract for cosmetic use made from mud, in particular from marine mud, **characterised in that** the following process steps are carried out:
- weighing the dried mud powder,
- addition of water and mixing the water and mud powder,
- addition of propylene glycol and glycerin,
- mixing the suspension for approximately 1 hour, slowly heating to approximately 60°C,
- cooling the mixture to room temperature,
- addition of ethanol and stirring the suspension for approximately 2 hours.

3. Method according to claim 2, **characterised in that**
- the mixture subsequently rests for approximately 24 hours, whereby a separation of the solid phase and the liquid phase is achieved.

4. Method according to claim 3, **characterised in that**
- the precipitated solid phase of the mixture is firstly coarse filtered and then fine filtered.

5. Method according to claim 4, **characterised in that** filter paper, in particular cellulose filter paper, is used for the fine filtration.

6. Method according to any of claims 1 to 5, **characterised in that** the ratio of the dried mud powder to the extractant is approximately 1:2 by mass.

## Revendications

1. Méthode de fabrication d'un extrait de boue, et en particulier de boue marine, à application cosmétique, **caractérisée en ce que** :
- la boue, contenant des organismes macroscopiques broyés, est mélangée sous forme d'une poudre déshydratée à un milieu d'extraction constitué de 5 à 15 parties pondérales d'eau, de 35 à 40 parties pondérales de glycérine, de 25 à 35 parties pondérales de propylène glycol et de 15 à 25 parties pondérales d'éthanol, la poudre de boue déshydratée constituant de 45 à 55 parties pondérales,
- le mélange du milieu d'extraction et de la poudre de boue étant agité à une température de 50°C à 70°C durant environ 1 à 3 heures et ensuite filtré.

2. Méthode de fabrication d'un extrait de boue, plus particulièrement de boue marine, à application cosmétique, **caractérisée en ce que** la méthode comporte les étapes de fabrication suivantes :
- pesée de la poudre de boue déshydratée ;
- adjonction d'eau à la poudre de boue et mélange ;
- adjonction de propylène glycol et de glycérine ;
- agitation de la suspension durant environ une heure et à feux doux à environ 60°C ;
- refroidissement du mélange jusqu'à température ambiante ;
- adjonction d'éthanol et agitation de la suspension durant environ deux heures.

3. Méthode selon la revendication 2, **caractérisée en ce que** :
- le mélange est ensuite laissé au repos durant environ 24 heures, ce qui conduit à une séparation des phases solide et liquide.

4. Méthode selon la revendication 3, **caractérisée en ce que** :
- la phase solide sédimentée du mélange est filtrée d'abord grossièrement et ensuite finement.

5. Méthode selon la revendication 4, **caractérisée en ce que** la filtration fine est effectuée avec un papier filtre, soit en particulier un papier filtre cellulosique.

6. Méthode selon l'une des revendications 1 à 5, **caractérisée en ce que** le rapport pondéral entre la poudre de boue déshydratée et le milieu d'extraction est d'environ 1 pour 2.
